# EUROPEAN PATENT APPLICATION

(11) **EP 1 876 619 A1**
(43) Date of publication of application: **09.01.2008**
(21) Application number: 06731316.3
(22) Date of filing: 06.04.2006
(51) Int. Cl.: H01H 13/64, H01H 23/30, G06F 3/02, H01H 3/46, G06F 3/033, A61B 6/03

(54) **MULTI-STAGE DETECTOR**

(30) Priority: 07.04.2005 JP 2005110665
(71) Applicant: Nemoto Kyorindo Co., Ltd., Tokyo 113-0033 (JP)
(72) Inventor: NEMOTO, Shigeru, Tokyo 1130033 (JP); FUKUDA, Takashi, Tokyo 1130033 (JP)
(74) Representative: Cabinet Plasseraud
(86) International application number: PCT/JP2006/307368
(87) International publication number: WO 2006/109691

(57) **Abstract**

A multi-stage detector capable of accurately detecting an input operations in steps. Since the vertical lengths of a plurality of displacement press members (137) and (138) displaceably supported on an up-and-down operation member (131) and downwardly energized by a press biasing mechanism (139) are different from each other. When the up-and-down operation member (131) is displaced downward, a plurality of press-contact detection elements (135) and (136) are pressed in order by the displacement press members (137) and (138).

## Description

### Technical Field

The present invention relates to a multi-stage detector for detecting a downward input operation in steps, and more particularly, to a multi-stage detector for use in an image display device.

### Background Art

Presently available imaging apparatuses in medical facilities for capturing successive tomographic images of a human body include CT (Computed Tomography) scanners and MRI (Magnetic Resonance Imaging) apparatuses. Various types of image display devices have been developed for displaying the image data provided by those apparatuses.

Such an image display device is realized, for example, by installing dedicated application software on a personal computer and allows a plurality of successive image data stored as series data to be shown in order on a display. In a typical personal computer, however, an input operation is performed through a keyboard including entry keys such as alphabetical keys and a mouse, so that the general versatility is excellent but the operability is poor.

To address this, an image display device proposed by the present applicant has a dedicated to-and-fro operation apparatus which has a to-and-fro operation member slidable forward and rearward. In the image display device, when the to-and-fro operation member is slid forward or rearward, image data shown on a display are accordingly switched to allow a user to easily view data of a plurality of successive tomographic images.

Particularly, in the image display device, slightly sliding the to-and-fro operation member from the initial position switches the displayed image data at a low speed, while largely sliding the to-and-fro operation member switches the displayed image data at a high speed. The user can intuitively adjust the switching speed of the image data flexibly.

In the to-and-fro operation member slid forward and rearward as described above, however, it is difficult to automatically return the to-and-fro operation member to the initial position after a manual operation is removed. Thus, when the plurality of displayed image data are sequentially switched, the user has difficulty in stopping the image switching at the instant when desired image data is displayed.

A to and fro operation apparatus capable of solving the problem is a product with a so-called seesaw switch. Such a to-and-fro operation apparatus includes a to-and-fro operation member elongated in the to-and-fro direction and supported at substantially the center to be pivotable forward and rearward. The to-and-fro operation member is biased to the initial position by the elastic force of an elastic member such as a spring.

When the to-andfro operation apparatus of that type is used in the abovementioned image display device, the to-and-fro operation member can be operated forward or rearward to switch displayed image data forward or rearward. Since the to-and-fro operation member is automatically returned to the initial position after the manual operation is removed, the image data can be immediately stopped at the desired position during the data switching for display.

Such a seesaw switch, however, can only detect the presence or absence of a forward or rearward input operation, so that it cannot adjust the speed at which the displayed image data are switched in the image display device. To adjust the speed at which the displayed image data are switched in the to-and-fro operation apparatus with the seesaw switch, it is necessary to provide a volume switch to adjust switching speed separately from the seesaw switch.

This is not preferable since the footprint of the apparatus is increased and the user needs to manually operate the two to-and-fro operation members at the same time. To address the abovementioned problems, various proposals have been made for a to and-fro operation apparatus which is operated in steps forward and rearward.

Such a to and fro operation apparatus can be realized as a configuration which includes a multi-stage detector for detecting a downward input operation in steps at each of positions where the front end and rear end of a to-and-fro operation member are pushed from above. A proposed example of the multi-stage detector in the to-and-fro operation apparatus includes a plurality of press-contact detection elements which require different pressures for press-contact and are arranged in an up-and-down direction (See, for example, Patent Document 1).

In another proposal, a plurality of press-contact detection elements require the same pressure for press-contact and support an operating plate which is pressed at positions displaced from the center thereof (See, for example, Patent Document 2). A yet another proposal includes a plurality of press-contact detection elements which require the same pressure for press-contact and are pressed uniformly through springs having different spring constants (See, for example, Patent Document 3). Another proposal includes a plurality of press-contact detection elements which require the same pressure for press-contact and are placed opposite in sets consisting of different numbers of the elements (See, for example, Patent Document 4).
Patent Document 1: Japanese Utility Model Publication No. 7-16269
Patent Document 2: Japanese Utility Model Publication No. 2-988
Patent Document 3: Japanese Utility Model Publication No. 2-131
Patent Document 4: Japanese Patent Laid-Open No. 2003-132'766
In Patent Document 1, to detect a downward input operation on the multi-stage detector in steps, the plurality of press-contact detection elements which are simultaneously pressed operate at different pressure levels. In the multi-stage detectors described in Patent Documents 2 to 4, the pressure in an input operation is applied to the plurality of press-contact detection elements unevenly or at unequal proportions.

In those multi-stage detectors, however, the pressure in an input operation is simultaneously applied to all of the press-contact detection elements in any case. Therefore age deterioration or manufacturing error may cause the plurality of press-contact detection elements to detect press contact for operation in different order from a desired order. It is difficult to reliably prevent erroneous detection.

### Disclosure of the Invention

The present invention has been made in view of the abovementioned problems, and it is an object thereof to provide a multi-stage detector capable of reliably detecting an input operation in steps with a simple configuration.

The multi-stage detector according to a first aspect of the present invention includes an up-and-down operation member, an operation support mechanism, displacement press members, a press biasing mechanism, press-contact detection elements, and a base member, and detects a downward input operation in steps. The operation support members supports the up-and-down operation member such that the up-and-down operation member is displaceable upward and downward. The up-and-down operation member receives the input operation. The n+1 displacement press members are supported on the up-and-down operation member such that the displacement press members are displaceable upward and downward. The press biasing mechanism biases each of the displacement press members downward against the up-and-down operation member. The n+1 press-contact detection elements detect press contact from above. The base member has the n+1 press-contact detection elements placed thereon at positions where the n+1 displacement press members are each opposed from above. Since the distances between the displacement press members and the opposed press-contact detection elements are different from each other in steps, a downward displacement of the up-and-down operation member causes the n+1 press-contact detection elements to be pressed by the n+1 displacement press members one by one.

The multi-stage detector according to a second aspect of the present invention also includes fixed press member, and n displacement press members are supported on an up-and-down operation member such that the displacement press members are displaceable upward and downward. The single fixed press member is fixed to the up-and-down operation member. A base member has n+1 press-contact detection elements placed thereon at positions where the n displacement press members and the single fixed press member are each opposed from above. Since the distances between the displacement/fixed press members and the opposed press-contact detection elements are different from each other in steps and the distance between the fixed press member and the press-contact detection element opposed thereto is the shortest, a downward displacement of the up-and-down operation member causes the n+1 press-contact detection elements to be pressed by the n displacement press members one by one and the finally by the fixed press member.

The multi-stage detector according to a third aspect of the present invention includes n+1 press-contact detection elements each placed at the lower ends of n+1 displacement press members and a base member opposed to the n+1 1 press-contact detection elements from below. Since the distances between the press-contact detection elements the opposed base member are different from each other in steps, a downward displacement of the up and down operation member causes the n+1 press-contact detection elements to be pressed by the base member one by one.

The multi-stage detector according to a fourth aspect of the present invention includes n+1 press-contact detection elements each placed at the lower ends of n displacement press members and at the lower end of a single fixed press member. Since the distances between the displacement/fixed press members and the opposed press-contact detection elements are different from each other in steps and the distance between the fixed press member and the base member opposed thereto is the longest, a downward displacement of the up-and-down operation member causes the n+1 press-contact detection elements to be pressed by the base member one by one.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of components may be constructed as one member, a certain component may be part of another component, or a certain component may have a portion overlapping a portion of another component.

Although the forward and rearward directions are specified in the present invention, these directions are defined for convenience to simply describe the relative relationship between components of the present invention and the definition does not limit any direction in manufacture or actual use when the present invention is implemented.

### Effect of the Invention

According to the multi-stage of the preset invention, a downward displacement of the up-and-down operation member causes the n+1 press-contact detection elements to be operated one by one. Thus, the stepwise input operation can be reliably detected with the simple configuration to prevent erroneous detection due to age deterioration or manufacturing error.

### Brief Description of the Drawings

Figs. 1(a) to 1(d) are front views showing the internal structure of a multi-stage detector of a to-and-front operation apparatus according to an embodiment of the present invention;
Fig. 2 is a side view showing the internal structure of the to-and-fro operation apparatus;
Fig. 3 is an exploded perspective view showing the assembly of the multi-stage detector;
Fig. 4 is a perspective view showing the structure of the multi-stage detector;
Fig. 5 is a plan view showing the outer appearance of a control unit;
Fig. 6 is a perspective view showing the outer appearance of an image display device;
Fig. 7 is a block diagram showing the circuit configuration of the image display device;
Fig. 8 is a perspective view showing the outer appearance of a tomography system;
Fig. 9 is a schematic diagram showing the logical configuration of the tomography system;
Fig. 10 is a front view showing image data displayed on a display unit;
Fig. 11 is a schematic diagram showing the data structure of series data;
Fig. 12 is a front view showing the internal structure of a multi-stage detector which is a first modification;
Fig. 13 is a front view showing the internal structure of a multi-stage detector which is a second modification;
Fig. 15 is a front view showing the internal structure of a multi-stage detector which is a third modification;
Fig. 16 is a front view showing the internal structure of a multi-stage detector which is a fourth modification; and
Fig. 17 is a front view showing the internal structure of a multi-stage detector which is a fifth modification.

### Description of Reference Numerals

- 100: TO-AND-FRO OPERATION APPARATUS
- 110, 167: BASE MEMBER
- 111: TO-AND-FRO SUPPORT MECHANISM
- 120: TO-AND-FRO OPERATION MEMBER SERVING AS SECOND OPERATION MEMBER
- 130, 150, 155, 160, 165, 170, 175: MULTI-STAGE DETECTOR
- 131, 151, 158, 171: UP-AND-DOWN OPERATION MEMBER
- 132: GUIDE SHAFT SERVING AS OPERATION SUPPORT MECHANISM
- 137, 138, 156, 166: DISPLACEMENT PRESS MEMBER
- 139: COIL SPRING SERVING AS PRESS URGING MECHANISM
- 134 TO 136, 157: PRESS-CONTACT DETECTION ELEMENT
- 152: FIXED PRESS MEMBER
- 300: IMAGE DISPLAY DEVICE
- 321: IMAGE ACQUIRING MEANS
- 322: IMAGE STORING MEANS
- 323: IMAGE DISPLAYING MEANS
- 324: IMAGE SWITCHING MEANS

### Best Mode for Carrying Out the Invention

### [Configuration of Embodiment]

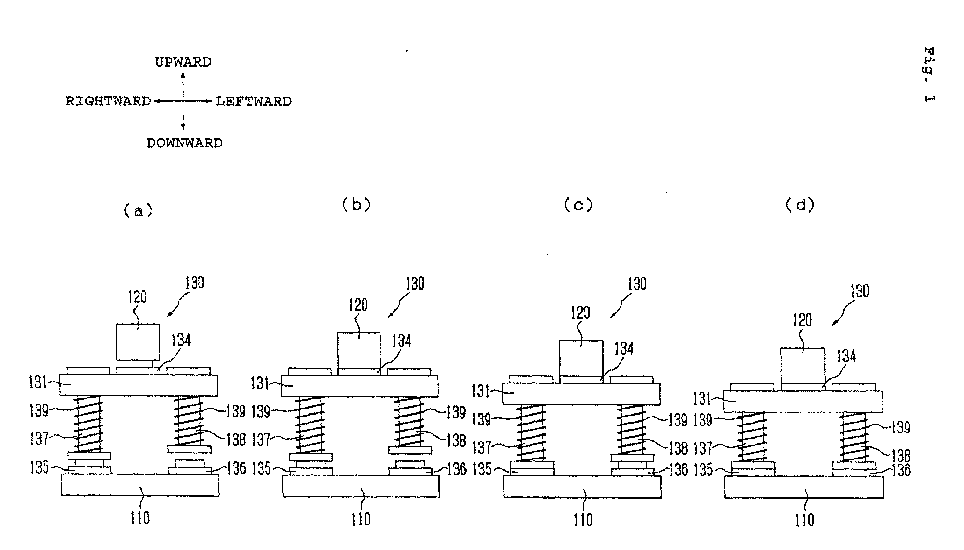

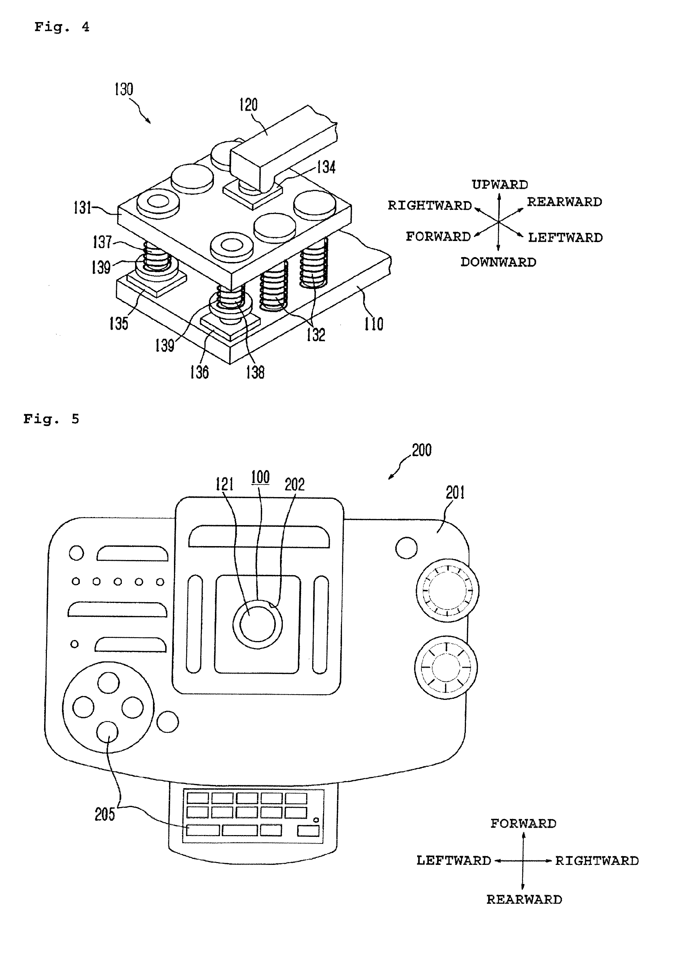

An embodiment of the present invention will hereinafter be described with reference to Figs. 1 to 17. To-and-fro operation apparatus 100 of the embodiment is formed as one unit part as shown in Figs. 1 to 4 and is contained in housing 201 of control unit 200 of image display device 300 as shown in Fig. 5.

To-and-fro operation apparatus 100 of the embodiment includes planar base member 110 elongated in a to-and-fro direction and to-and-fro operation member 120. To-and-fro support mechanisms 111 provided to stand on the right and left at the center of base member 110 support to-and-fro operation member 120 serving as a second operation member, to be pivotable forward and rearward on the center.

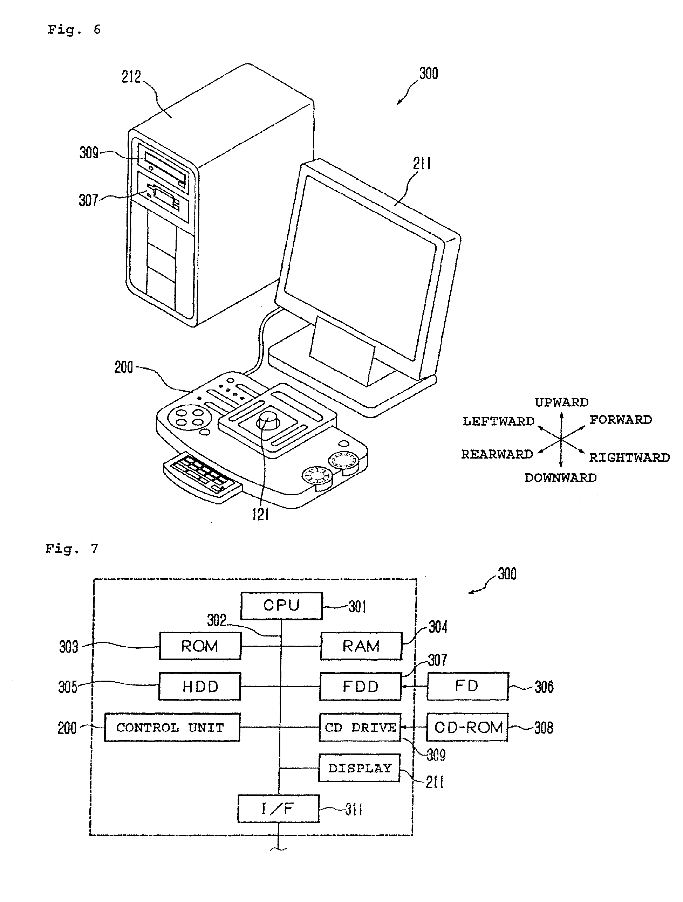

Joystick 121 is provided to stand at the center of the upper surface of to and fro operation member 120, and protrudes upward from through hole 202 of control unit 200 as shown in Figs. 5 and 6. Each of end portions 122 in the front and rear of to-and-fro operation member 120 is formed in a semi-cylindrical shape protruding downward. Multi-stage detectors 130 are provided, as a front switch and a rear switch, at the positions where they are pushed from above by end portions 122 in the front and rear.

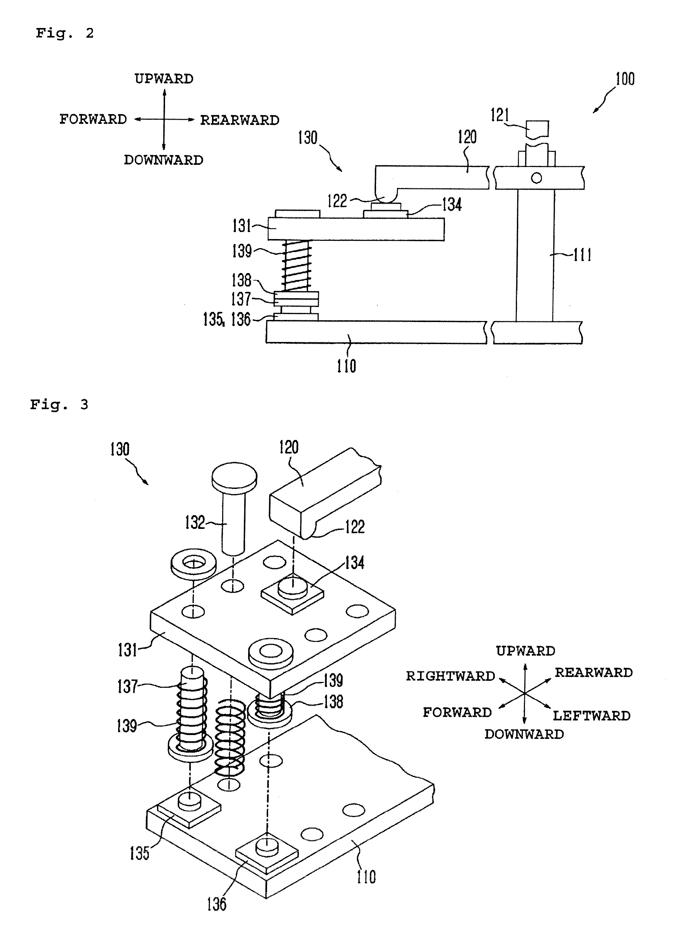

Multi-stage detector 130 has up-and-down operation member 131 with planar and rectangular shape, to which end portion 122 of to-and-fro operation member 120 is opposed from above. As shown in Figs. 3 and 4, two pairs of guide shafts 132 as operation support members are provided to stand on the upper surface of base member 110 in the front and rear end portions. Guide shafts 132 support up-and-down operation member 131 to be slidable upward and downward.

A coil spring (not shown) is wound on each guide shaft 132 and it serves as an operation biasing mechanism, to elastically bias up-and-down operation member 131 upward. Since upper end of each guide shaft 132 is formed as a stopper extending outward, up-and-down operation member 131 is held at the positions of the upper ends of guide shafts 132 such that it is biased upward and is slidable upward and downward.

Two, or n+1, press-contact detection elements 135 and 136 are placed in the front and rear ends on the upper surface of base member 110, such that one of them is located on the left and the other on the right. Two, or n+1, displacement press members 137 and 138 are slidably mounted on up-and-down operation member 131 at the positions where they are opposed to press-contact detection elements 135 and 136 from above, respectively.

Coil spring 139 is wound on each of displacement press members 137 and 138, and serves as a press biasing mechanism, to bias displacement press members 137 and 138 downward against up-and-down operation member 131. Since upper end of each of displacement press members 137 and 138 is formed as a stopper extending outward, their lower ends are opposed to press-contact detection elements 135 and 136, while they are biased downward and slidable upward and downward.

However in multi-stage detector 130 of the embodiment, the vertical lengths of two displacement press members 137 and 138 that protrude downward from up-and-down operation member 131 are different from each other in steps. Therefore the distances between displacement press members 137 and 138 and opposed press-contact detection elements 135 and 136 are different. In multi-stage detector 130 of the embodiment, third, or n+2th, press-contact detection element 134 is placed on the upper surface of up and down operation member 131, and end portion 122 of to and fro operation member 120 is opposed to press-contact detection element 134 from above.

Each of press-contact detection elements 134 to 136 is formed such that element displacement member 142 is supported to be elastically retractable on detection element body 141. Press-contact detection elements 134 to 136 require the same pressure for operation. However, up-and-down operation member 131 is biased upward by the coil spring as described above, so that a downward displacement of end portion 122 of to-and-fro operation member 120 first presses only press-contact detection element 134 on the upper surface of up-and-down operation member 131, as shown in Figs. 1(a) and 1(b).

When end portion 122 of to-and-fro operation member 120 is further displaced downward, longer displacement press member 137 presses press-contact detection element 135 on the upper surface of base member 110 as shown in Fig. 1(c). When end portion 122 of to-and-fro operation member 120 is further displaced downward, shorter displacement press member 138 presses the other press-contact detection element 136 as shown in Fig. 1(d).

As shown in Fig. 5, control unit 200 of the embodiment has through hole 202 at the center of the upper surface of housing 201. Joystick 121 of to-and-fro operation member 100 is exposed upward from through hole 202. Control unit 200 has various operation keys 205 mounted thereon in addition to to-and-fro operation apparatus 100. Operation keys 205 and to-and-fro operation apparatus 100 are connected to a circuit board (not shown).

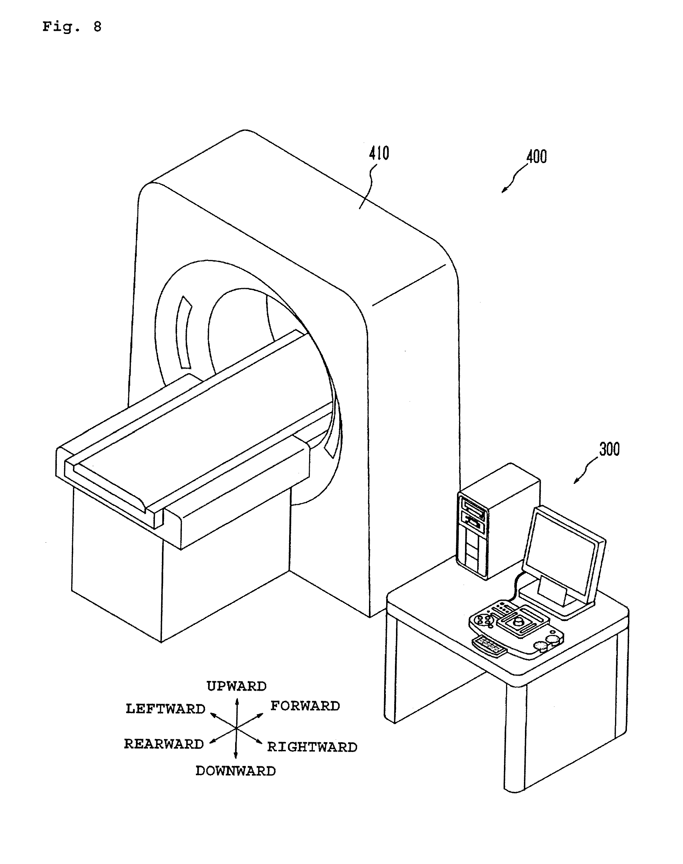

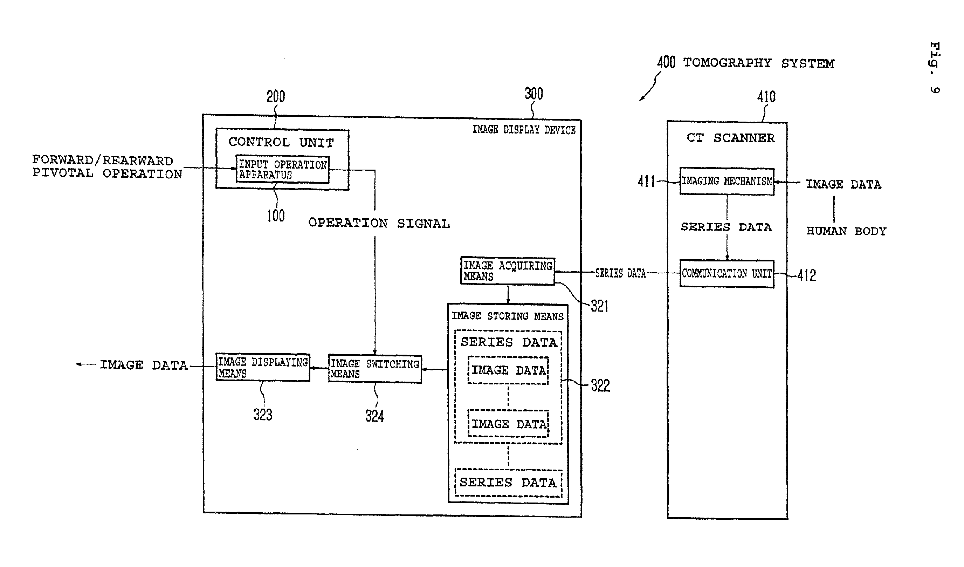

As shown in Figs. 6 and 7, control unit 200 of the embodiment is formed as part of image display device 300, and is connected to body unit 212 together with display unit 211. As shown in Figs. 8 and 9, image display device 300 of the embodiment is formed as part of tomography system 400, and is online connected to CT scanner 410.

Image display device 300 of the embodiment includes CPU (Central Processing Unit) 301 as hardware which is a main part of a computer. CPU 301 is connected through bus line 302 to pieces of hardware such as ROM (Read Only Memory) 303, RAM (Random Access Memory) 304, HDD 305 (Hard Disc Drive) 305, FDD (Flexible Disc Drive) 307 on which FD (Flexible Disc-cartridge) 306 is replaceably loaded, CD (Compact Disc) drive 309 on which CD-ROM 308 is replaceably loaded, control unit 200, display unit 211, and I/F (Interface) unit 311.

CT scanner 410 includes imaging mechanism 411 serving as an imaging means and communication unit 412 serving as an image supply means. Imaging mechanism 411 takes image data of a plurality of successive tomographic images from a human body (not shown). A plurality of image data successively taken from a single human body are accumulated as series data. Communication unit 412 transmits the data to image display device 300.

In image display device 300 of the embodiment, pieces of hardware such as ROM 303, RAM 304, HDD 305, replaceable FD 306, and replaceable CD-ROM 308 correspond to information storage media, and computer programs or resources for CPU 301 are stored as software on at least one of them. Such software is previously installed on image display device 300 and is read as data by CPU 301 when image display device 300 is started up.

CPU reads the data of the appropriate computer program to execute various processing to cause image display device 300 of the embodiment to logically have, as various functions, various means such as image acquiring means 321, image storing means 322, image displaying means 323, and image switching means 324 as shown in Fig. 9.

Image acquiring means 321 corresponds to the function of CPU 301 which recognizes data received by I/F unit 311 in accordance with the computer program stored on RAM 304 and acquires the series data from CT scanner 410. Image storing means 322 corresponds to the storage area created on HDD 305 to be recognized by CPU 301 in accordance with the abovementioned computer program and stores the series data acquired by image acquiring means 321.

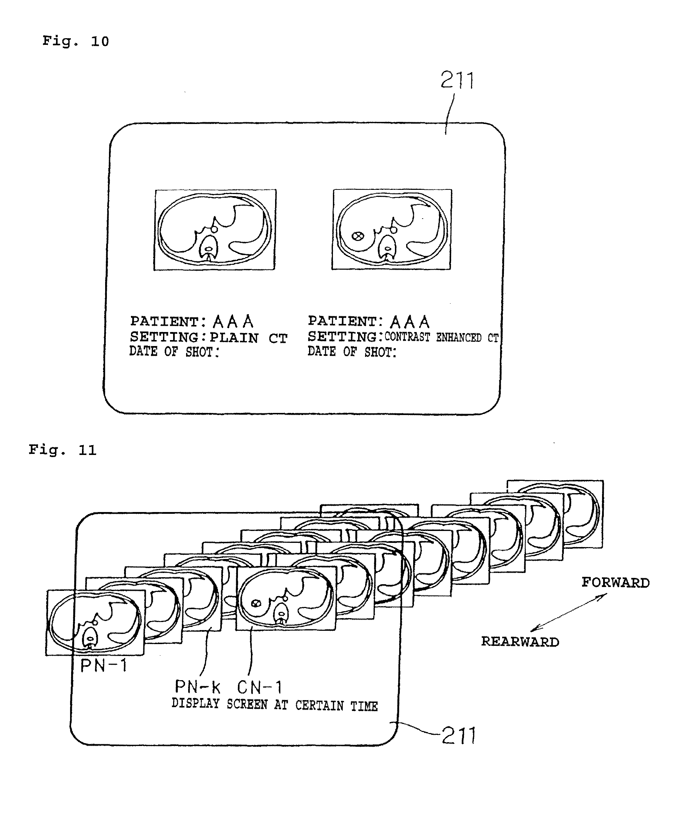

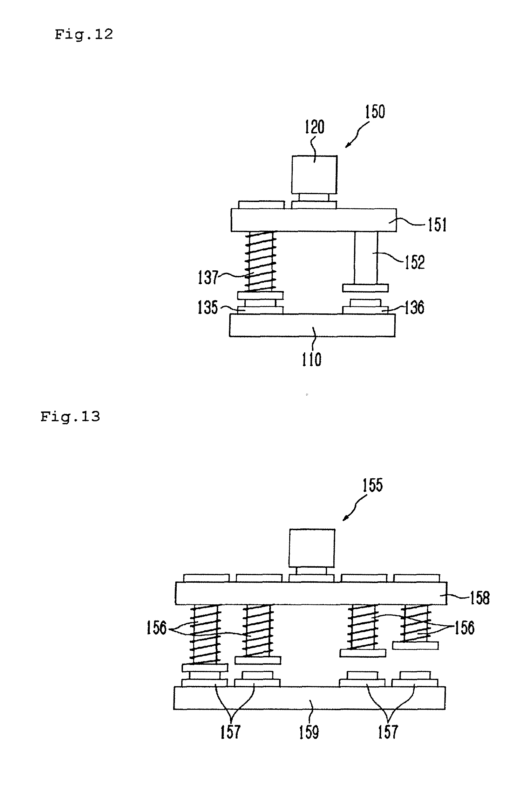

Image displaying means 323 corresponds to the function of CPU 301 which displays on display unit 211 the data stored on HDD 305 and displays at least one image data from the series data stored in image storing means 322. The display can be performed in various forms, and in image display device 300 of the embodiment, the image data of the associated pair of series data can be displayed side by side, as shown in Figs. 10 and 11 by performing a predetermined operation with operation keys 205 of control unit 200.

Image switching means 324 corresponds to the function of CPU 301 which recognizes an operation signal from control unit 200 to perform predetermined data processing, and switches the image data displayed by image displaying means 323 in accordance with an input operation on to-and-fro operation member 120 of control unit 200.

More specifically, in image display device 300 of the embodiment, when joystick 121 of to-and-fro operation apparatus 100 is inclined forward at a predetermined angle with a predetermined pressure to press only press-contact detection element 134 on the upper surface of up-and-down operation member 131 of multi-stage detector 130 in the front, only one image data of the series data displayed is switched forward in response. When joystick 121 is inclined rearward to press only press-contact detection element 134 of multi-stage detector 130 in the rear, only one image data is switched rearward in response.

Then, when joystick 121 is further inclined forward or rearward to cause longer displacement press member 137 of multi-stage detector 130 in the front or rear to press press-contact detection element 135 on the upper surface of base member 110, the displayed image data of the series data is successively switched at a low speed forward or rearward.

Then, when joystick 121 is further inclined forward or rearward to cause shorter displacement press member 138 of multi-stage detector 130 in the front or rear to press the other press-contact detection element 136, the displayed image data of the series data is successively switched at a high speed forward or rearward.

In image display device 300 of the embodiment, in addition to the basic operation for displaying one image data of one series data on display unit 211, a plurality of image data can be displayed side by side as an application operation as shown in Figs. 10 and 11. In this display state, when a predetermined operation is performed through operation key 205 of to-and-fro operation apparatus 100, one of the plurality of image data displayed side by side is selected for forward or rearward switching with to-and-fro operation apparatus 100.

Although the abovementioned various means of image display device 300 are accomplished by pieces of hardware such as HDD 305 and I/F unit 311 as required, they are mainly implemented by CPU 301 as a piece of hardware functioning in accordance with the computer programs stored on the information storage medium such as RAM 304.

The computer programs are stored on the information storage medium such as RAM 304 as software for causing CPU 301 or the like to perform processing operations including the reception of the series data by I/F unit 311 from CT scanner 410, the storage of the received series data on HDD 305, the display of the image data of the stored series data on display unit 211, and the forward or rearward switching of the displayed image data in accordance with the output signal from to-and-fro operation apparatus 100 of control unit 200.

### [Operation of the Embodiment]

In the abovementioned configuration, the test method in tomography system 400 of the embodiment will hereinafter be described in order. First, a plurality of successive tomographic image data are taken from the body of a patient (not shown) by CT scanner 410 and are registered as series data in image display device 300.

After the series data are registered in image display device 300 in this manner, an operator (not shown) operates control unit 200 to display the image data of the series data on display unit 211. With the image data displayed, joystick 121 of control unit 200 is operated for input. In response thereto, the displayed image data is manipulated.

More specifically, in image display device 300 of the embodiment, when joystick 121 of to-and-fro operation apparatus 100 is inclined forward or rearward at a predetermined angle with a predetermined pressure during the display of the image data of one series data on display unit 211, only press-contact detection element 134 on the upper surface of up-and-down operation member 131 of multi-stage detector 130 in the front or rear is pressed as shown in Figs. 1(a) and 1(b). In response thereto, the displayed image data of the series data is switched forward or rearward to the next one. Each time this input operation is performed, the displayed image data of the series data is switched forward or rearward one by one.

Then, joystick 121 is further inclined forward or rearward at a predetermined angle with a predetermined pressure to cause longer displacement press member 137 of multi-stage detector 130 in the front or rear to press press-contact detection element 135 on the upper surface of base member 110 as shown in Fig. 1(c). In response thereto, the displayed image data of the series data is switched successively forward or rearward at a low speed.

Then, joystick 121 is further inclined to cause shorter displacement press member 138 of multi-stage detector 130 in the front or rear to press the other press-contact detection element 136 as shown in Fig. 1(d). In response thereto, the displayed image data of the series data is switched successively forward or rearward at a high speed.

In this manner, in image display device 300 of the embodiment, each time joystick 121 is slightly moved forward or rearward, the displayed image data is switched forward or rearward one by one. Whereas Each time joystick 121 is significantly moved forward or rearward, the displayed image data is successively switched forward or rearward at a speed in accordance with the input stress and the inclination angle.

In image display device 300 of the embodiment, in addition to the display of image data of the series data one by one on display unit 211 as described above, the image data of a plurality of series data can be displayed side by side as shown in Figs. 10 and 11. For example, series data are captured and registered from a patient both when a contrast medium is used and when no contrast medium is used, and the pair of series data can be displayed side by side.

In image display device 300 of the embodiment, when a predetermined operation is performed through operation keys 205 of to-and-fro operation apparatus 100 in the abovementioned display state, one of the plurality of image data displayed side by side is selected for forward and rearward switching with to-and-fro operation apparatus 100.

### [Effect of the Embodiment]

In image display device 300 of the embodiment, the input operation of joystick 121 of to-and-fro operation apparatus 100 as described above allows the various manipulation of the displayed image data of the series data. Particularly, since joystick 121 can be operated in the three stages forward and rearward, the image data of the series data can be switched one by one forward and rearward. The image data of the series data can be successively switched at the low and high speeds, and those switching operations can be performed intuitively.

In image display device 300 of the embodiment, since the forward and rearward operations of joystick 212 in the three stages can be accurately detected by multi-stage detector 130, the switching operation of the image data can be accurately performed.

Specifically, in multi-stage detector 130 of the embodiment, although press-contact detection elements 134 to 136 require the same pressure for operation, since only pres-contact detection element 134 is mounted on up-and-down operation member 131 biased upward by the coil spring, when a downward displacement of end portion 122 of to-and-fro operation member 120 as shown in Figs. 1(a) and 1(b), first, only press-contact detection element 134 on the upper surface of up-and-down operation member 131 is pressed. At this point, since the pressure on press-contact detection element 134 from to-and-fro operation member 120 is not applied to press-contact detection elements 135 or 136 on the upper surface of base member 110, press-contact detection elements 135 and 136 do not erroneously detect the pressure.

In multi-stage detector 130 of the embodiment, since the vertical lengths of displacement press members 137 and 138 that protrude downward from up-and-down operation member 131 are different from each other, when end portion 122 of to-and-fro operation member 120 is further displaced downward from the abovementioned state, press-contact detection element 135 on the upper surface of base member 110 is pressed by longer displacement press member 137 as shown in Fig. 1(c). At this point, since the pressure is not applied to the other press-contact detection element 136, press-contact detection element 136 does not erroneously detect the pressure.

Then, when end portion 122 of to-and-fro operation member 120 is further displaced downward, the other press-contact detection element 136 is pressed by shorter displacement press member 138 as shown in Fig. 1(d). Thus, multi-stage detector 130 of the embodiment can reliably detect the three-stage input operations to favorably prevent erroneous detection due to age degradation or manufacturing error.

Particularly, displacement press members 137 and 138 are slidably supported by up-and-down operation member 131 so that the press contact between longer displacement press member 137 and press-contact detection element 135 does not obstruct the press contact between shorter displacement press member 138 and press-contact detection element 136. Since displacement press members 137 and 138 are displaced downward by coil spring 138 against up-and-down operation member 131, displacement press members 137 and 138 can favorably apply the pressure to press-contact detection members 135 and 136.

In multi-stage detector 130 of the embodiment, three press-contact detection elements 134 to 136 for detecting the three-stage input operations as described above can require the same pressure for operation, therefore press-contact detection elements 134 to 136 can realized by the same existing product to achieve excellent productivity of multi-stage detector 130.

### [Modifications of the Embodiment]

The present invention is not in any way limited to the abovementioned embodiment, but various changes and modifications may be made therein without departing from the scope of the invention. For example, in the embodiment, to and fro operation apparatus 100 is formed of two multi-stage detectors 130 to detect the forward and rearward input operations. However one multi-stage detector 130 may be used to detect a downward input operations in multiple stages, or an input operation apparatus (not shown) formed of four multi-stage detectors 130 placed in the front, rear, left and right may be used to detect forward, rearward, leftward and rightward input operations in multiple stages.

Multi-stage detector 130 of the embodiment includes not only n+1 press-contact detection elements 135 and 136 on base member 110 but also press-contact detection element 134 on the upper surface of up-and-down operation member 131. However, this may be omitted as in multi-stage detector 150 shown in Fig. 12. In such multi-stage detector 150, up-and-down operation member 131 may be directly operated manually by integrally forming to-and-fro operation member 120 together with up-and-down operation member 131.

In multi-stage detector 130 of the embodiment, all of n+1 displacement press members 137 and 138 are displaceably mounted on up-and-down operation member 131. However as shown in Fig. 12, the shortest displacement press member may be fixed as fixed press member 152 to up-down operation member 151.

In multi-stage detector 130 of the embodiment, two displacement press members 137 and 138 are mounted on up-and-down operation member 131 and two press-contact detection elements 135 and 136 are mounted on base member 110. However as in multi-stage detector 155 shown in Fig. 13, three or more displacement press members 156 and three or more press-contact detection elements 157 may be mounted on up-down operation member 158 and base member 159, respectively.

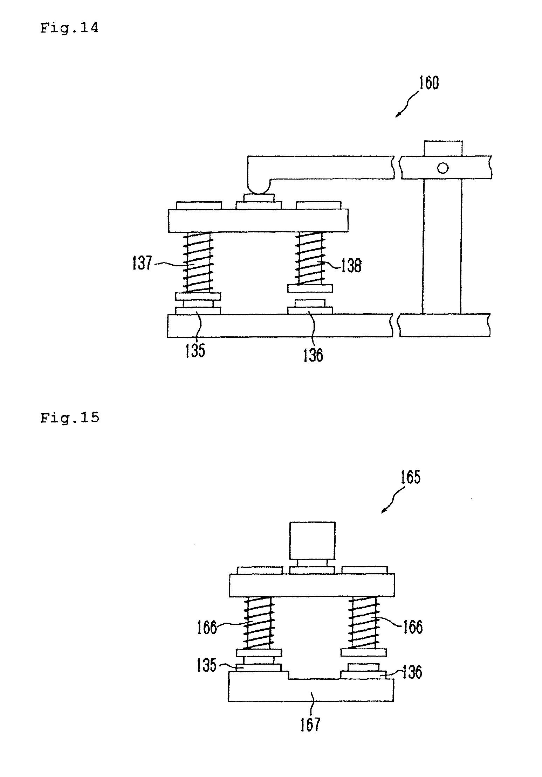

In multi-stage detector 130 of the embodiment, displacement press members 137 and 138 and press-contact detection elements 135 and 136 are arranged in the left-to-right direction. However as in multi-stage detector 160 shown in Fig. 14, displacement press members 137 and 138 and press-contact detection elements 135 and 136 may be arranged in the to-and-fro direction. For example, four displacement press members and four press-contact detection elements may be arranged in the front, rear, left and right in a rectangular (not shown).

In multi-stage detector 130 of the embodiment, the distances between press-contact detection elements 135 and 136 and opposed displacement press members 137 and 138 are different in steps in order to operate press-contact detection elements 135 and 136 in steps. To realize the distances different in steps, the vertical lengths of displacement press members 137 and 138 that protrude downward from up-and-down operation member 131 are different from each other in steps.

However as in multi-stage detector 165 shown in Fig. 15, the vertical lengths of n+1 displacement press members 166 that protrude downward from up-and-down operation member 131 may be the same, and base member 167 having press-contact detection elements 135 and 136 placed thereon may have n+1 upper surfaces which are at different heights in steps.

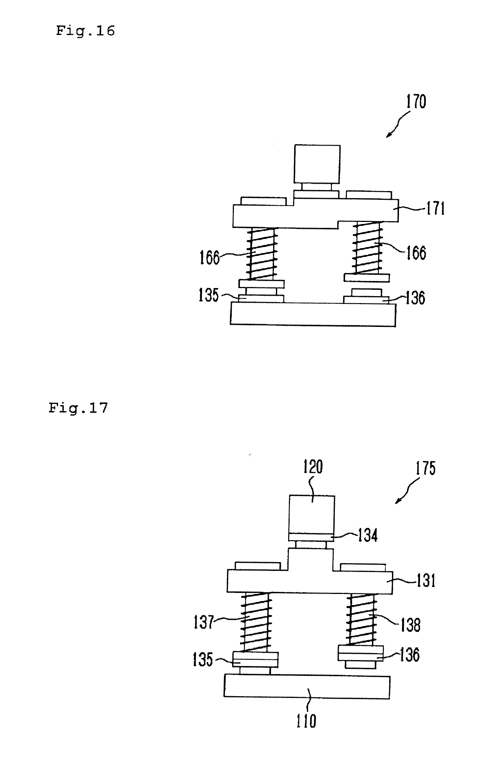

As in multi-stage detector 170 shown in Fig. 16, the vertical lengths of n+1 displacement press members 166 that protrude downward from up-and-down operation member 171 may be the same, and up-and-down operation member 171 supporting displacement press members 166 may have n+1 surfaces which are at different heights in steps.

In multi-stage detector 130 of the embodiment, press-contact detection elements 135 and 136 are placed on the upper surface of base member 110, and displacement press members 137 and 138 are opposed to press-contact detection elements 135 and 136 from above. However as in multi-stage detector 175 shown in Fig. 17, press-contact detection elements 135 and 136 mounted on the lower surfaces of displacement press members 137 and 138 may be opposed to base member 110 from above.

In multi-stage detector 130 of the embodiment, to-and-fro operation member 120 is opposed from above to press-contact detection element 134 mounted on the upper surface of up-and-down operation member 131. However press-contact detection element 134 mounted on the lower surface of to-and-fro operation member 120 may be opposed to up-and-down operation member 131 from above.

In multi-stage detector 130 of the embodiment, up-and-down operation member 131 is biased upward by the operation biasing mechanism made of the dedicated coil spring. However for example, part or the entire of the operation biasing member may be formed of coil spring 139 which biases each of displacement press member 137 and 138 downward, or, element displacement member 142 elastically supported on detection element body 141 of each of press-contact detection elements 135 and 136.

In the embodiment, to-and-fro operation member 120 of to-and-fro operation apparatus 100 is manually operated with fingers by assuming a proper size for to-and-fro operation member 120. However for example, to-and-fro operation member 120 may be increased in size to allow an input operation with a foot. It goes without saying that the abovementioned various modifications may be combined flexibly such that no contradiction occurs.

In image display device 300 of the embodiment, image display device 300 displays a pair of image data side by side on display unit 211. However naturally, only one image data may be displayed or two or more image data can be displayed.

In image display device 300 of the embodiment, image data of a plurality of series data are displayed side by side. However for example, a plurality of image data at different positions forward and rearward in one series data may be displayed side by side, or, one image data can be displayed at a plurality of positions with different image processing.

In the embodiment, the image data displayed on display unit 211 by image display device 300 is switched with one to-and-fro operation apparatus 100 of control unit 200. However for example, it is possible to provide a plurality of to-and-fro operation apparatuses 100 for control unit 200 such that a plurality of image data displayed side by side may be switched individually. Moreover though the series data displayed in image display device 300 taken by CT scanner 410 in the embodiment, the image data may be taken by an MRI apparatus (not shown).

In the embodiment, CPU 301 operates in accordance with the computer program stored in RAM 304 or the like to realize logically various means as various functions of image display device 300. However each of the various means may be formed as specific hardware, or some of them may be stored as software in RAM 304 or the like, while others may be formed as hardware.

## Claims

1. A multi-stage detector for detecting a downward input operation in steps, comprising:
an up-and-down operation member receiving the input operation;
an operation support mechanism supporting the up-and-down operation member such that the up-and-down operation member is displaceable upward and downward;
n+1 (where n means a natural number) displacement press members supported on the up-and-down operation member such that the displacement press members are displaceable upward and downward;
a press biasing mechanism biasing each of the displacement press members downward against the up-and-down operation member;
n+1 press-contact detection elements detecting press contact from above; and
a base member on which the n+1 press-contact detection elements are placed at positions where the n+1 displacement press members are each opposed from above,
wherein the distances between the displacement press members and the opposed press-contact detection elements are different from each other in steps.

2. A multi-stage detector for detecting a downward input operation in steps, comprising:
an up-and-down operation member receiving the input operation;
an operation support mechanism supporting the up-and-down operation member such that the up-and-down operation member is displaceable upward and downward;
n (where n means a natural number) displacement press members supported on the up-and-down operation member such that the displacement press members are displaceable upward and downward;
a press biasing mechanism biasing each of the displacement press members downward against the up-and-down operation member;
a single fixed press member fixed to the up-and-down operation member;
n+1 press-contact detection elements each detecting press contact from above; and
a base member on which the n+1 press-contact detection elements are placed at positions where the n displacement press members and the single fixed press member are each opposed from above,
wherein the distances between the displacement/fixed press members and the opposed press-contact detection elements are different from each other in steps and the distance between the fixed press member and the press-contact detection element opposed thereto is the longest.

3. A multi-stage detector for detecting a downward input operation in steps, comprising:
an up-and-down operation member receiving the input operation;
an operation support mechanism supporting the up-and-down operation member such that the up-and-down operation member is displaceable upward and downward;
n+1 (where n means a natural number) displacement press members supported on the up-and-down operation member such that the displacement press members are displaceable upward and downward;
a press biasing mechanism biasing each of the displacement press members downward against the up and down operation member;
n+1 press-contact detection elements each placed at lower ends of the displacement press members and detecting press contact from below; and
a base member opposed to the n+1 press-contact detection elements from below,
wherein the distances between the press-contact detection elements and the opposed base member are different from each other in steps.

4. A multi-stage detector for detecting a downward input operation in steps, comprising:
an up-and-down operation member receiving the input operation;
an operation support mechanism supporting the up-and-down operation member such that the up-and-down operation member is displaceable upward and downward;
n (where n means a natural number) displacement press members supported on the up-and-down operation member such that the displacement press members are displaceable upward and downward;
a press biasing mechanism biasing each of the displacement press members downward against the up-and-down operation member;
a single fixed press member fixed to the up-and-down operation member;
n+1 press-contact detection elements each placed at lower ends of the n displacement press members and at a lower end of the single fixed press member and detecting press contact from below; and
a base member opposed to the n+1 press-contact detection elements from below,
wherein the distances between the displacement/fixed press members and the opposed press-contact detection elements are different from each other in steps and the distance between the fixed press member and the base member opposed thereto is the longest.

5. The multi-stage detector according to claim 1 or 3, wherein the vertical lengths of the n+1 displacement press members that protrude downward from the up-and-down operation member are different from each other in steps.

6. The multi-stage detector according to claim 2 or 4, wherein and the vertical lengths of the n displacement press members and the single fixed press member that protrude downward from the up-and-down operation member are different from each other in steps and the vertical length of the fixed press member that protrudes downward from the up-and-down operation member is the shortest.

7. The multi-stage detector according to claim 1 or 3, wherein n+1 positions of the up-and-down operation member where the displacement press members are supported are different in a vertical direction from each other in steps.

8. The multi-stage detector according to claim 2 or 4, wherein n positions of the up-and-down operation member where the displacement press members are supported are different in a vertical direction from each other in steps, and
the lower end of the fixed press member is higher than the lower end of the uppermost displacement press member.

9. The multi-stage detector according to claim 1 or 2, wherein the positions of n+1 upper surfaces of the base member where the press-contact detection elements are each placed are different in a vertical direction from each other in steps.

10. The multi-stage detector according to claim 3 or 4, wherein positions of n+1 upper surfaces of the base member where the press-contact detection elements are each opposed are different in a vertical direction from each other in steps.

11. The multi-stage detector according to any one of claims 1 to 10, further comprising an operation biasing mechanism biasing the up-and-down operation member upward.

12. The multi-stage detector according to claim 11, wherein at least part of the operation biasing mechanism is formed of the press biasing mechanism.

13. The multi-stage detector according to claim 11 or 12, wherein the press-contact detection element includes a detection element body and an element displacement member supported to be retractable elastically on the detection element body, and
at least part of the operation biasing mechanism is formed of the press-contact detection element.

14. The multi-stage detector according to any one of claims 1 to 13, further comprising:
a second operation member placed at a position where it faces to the up-and-down operation member from above;
a second support mechanism supporting the second operation member such that the second operation member is displaceable upward and downward; and
an n+2th press-contact detection element placed on an upper surface of the up and down operation member at a position where the second operation member is opposed from above.

15. The multi-stage detector according to any one of claims 1 to 13, further comprising:
a second operation member placed at a position where it faces to the up-and-down operation member from above;
a second support mechanism supporting the second operation member such that the second operation member is displaceable upward and downward; and
an n+2th press-contact detection element placed on a lower surface of the second operation member at a position where the up-and-down operation member is opposed from below.

16. A to-and-fro operation apparatus for receiving a forward and rearward input operation in steps, comprising:
a to-and-fro operation member receiving the input operation;
a forward switch formed of the multi-stage detector according to any one of claims 1 to 13, wherein the up-and-down operation member is pressed downward when the to-and-fro operation member is forward operated for input; and
a rearward switch formed of the multi-stage detector according to any one of claims 1 to 13, wherein the up-and-down operation member is pressed downward when the to-and-fro operation member is rearward operated for input.

17. A to-and-fro operation apparatus for receiving a forward and rearward input operation in steps, comprising
a to-and-fro operation member receiving the input operation;
a forward switch formed of the multi-stage detector according to claim 14 or 15, wherein the second operation member is pressed downward when the to and fro operation member is forward operated for input; and
a rearward switch formed of the multi-stage detector according to claim 14 or 15, wherein the second operation member is pressed downward when the to-and-fro operation member is rearward operated for input.

18. The to-and-fra operation apparatus according to claim 17, wherein a front end of the to-and-fro operation member is integrally formed with the second operation member of the forward switch, and
a rear end of the to-and-fro operation member is integrally formed with the second operation member of the forward switch.

19. The to and fro operation apparatus according to any one of claims 16 to 18, wherein the to and fro operation member supported to be inclinable forward and rearward is elastically biased to the center by the forward switch and the rearward switch.

20. An image display device comprising:
the to-and-fro operation apparatus according to any one of claims 16 to 19;
image storing means for storing at least one series data formed of a plurality of successive image data;
image displaying means for displaying at least one of the image data of the stored series data; and
image switching means for switching the image data forward at a stepwise variable speed when the forward switch detects in steps a forward inclination of the to-and-fro operation member of the to-and-fro operation apparatus, and for switching the image data rearward at a stepwise variable speed when the rearward switch detects in steps a rearward inclination of the to and fro operation member of the to and fro operation apparatus.

21. An image display device comprising:
the to-and-fro operation apparatus according to claim 16;
image storing means for storing at least one series data formed of a plurality of successive image data;
image displaying means for displaying at least one of the image data of the stored series data; and
image switching means for switching the displayed image data forward and rearward when the to-and-fro operation member of the to-and-fro operation apparatus is inclined forward and rearward,
wherein the image switching means switches the image data forward one by one each time the to-and-fro operation member of the to-and-fro operation apparatus is inclined forward in a predetermined range to cause a first one of the press-contact detection elements of the forward switch to detect press contact, switches the image data rearward one by one each time the to-and-fro operation member is inclined rearward in a predetermined range to cause a first one of the press-contact detection elements of the rearward switch to detect press contact, switches the image data forward at a variable speed in n stages each time the to and fro operation member is inclined forward in steps beyond the predetermined range to cause a second one to an n+1th one of the press-contact detection elements of the forward switch to detect press contact, and switches the image data rearward at a variable speed in n stages each time the to-and-fro operation member is inclined rearward in steps beyond the predetermined range to cause a second one to an n+1th one of the press-contact detection elements of the rearward switch to detect press contact.

22. An image display device comprising:
the to and fro operation apparatus according to claim 17 or 18;
image storing means for storing at least one series data formed of a plurality of successive image data;
image displaying means for displaying at least one of the image data of the stored series data; and
image switching means for switching the displayed image data forward and rearward when the to-and-fro operation member of the to-and-fro operation apparatus is inclined forward and rearward,
wherein the image switching means switches the image data forward one by one each time the to-and-fro operation member of the to-and-fro operation apparatus is inclined forward in a predetermined range to cause an n+2th one of the press-contact detection elements of the forward switch to detect press contact, switches the image data rearward one by one each time the to-and-fro operation member is inclined rearward in a predetermined range to cause an n+2th one of the press contact detection elements of the rearward switch to detect press contact, switches the image data forward at a variable speed in n stages each time the to and fro operation member is inclined forward in steps beyond the predetermined range to cause a second one to an n+1th one of the press-contact detection elements of the forward switch to detect press contact, and switches the image data rearward at a variable speed in n stages each time the to-and-fro operation member is inclined rearward in steps beyond the predetermined range to cause a second one to an n+1th one of the press-contact detection elements of the rearward switch to detect press contact.
